Europäisches Patentamt

European Patent Office

Office européen des brevets

(19)

(11) Publication number: **0 455 042 A1**

# EUROPEAN PATENT APPLICATION

(12)

(21) Application number: **91106054.9**

(22) Date of filing: **16.04.91**

(51) Int. Cl.⁵: **A61K 31/19**, A61K 31/365,
//(A61K31/365,31:19)

(30) Priority: **30.04.90 US 516865**

(43) Date of publication of application:
**06.11.91 Bulletin 91/45**

(84) Designated Contracting States:
**AT BE CH DE DK ES FR GB GR IT LI LU NL SE**

(71) Applicant: **E.R. SQUIBB & SONS, INC.**
**P.O.Box 4000**
**Princeton New Jersey 08543-4000(US)**

(72) Inventor: **Pan, Henry Y.**
**24 Cartwright Drive West**
**Princeton Junction, NJ 08550(US)**

(74) Representative: **Josif, Albert et al**
**Baaderstrasse 3**
**W-8000 München 5(DE)**

(54) Combination of pravastatin and a fibric acid derivative, and method for treating dyslipidemia using such combination.

(57) A pharmaceutical combination is provided which includes pravastatin and a fibric acid derivative, such as fenofibrate, gemfibrozil or bezafibrate. A method for treating dyslipidemia in non-diabetics and diabetics using the above combination is also provided.

EP 0 455 042 A1

The present invention relates to a combination of an inhibitor of 3-hydroxy-3-methyl-glutaryl coenzyme A (HMG CoA) reductase which is pravastatin and a derivative of fibric acid, and to a method for treating dyslipidemia in diabetics and non-diabetics by administering such combination.

There are several different classes of compounds which have serum cholesterol lowering properties. Some of these compounds are inhibitors of the enzyme HMG CoA reductase which is essential in the production of cholesterol, such as mevastatin (disclosed in U. S. Patent No. 3,983,140), lovastatin also referred to as mevinolin (disclosed in U. S. Patent No. 4,231,938), pravastatin (disclosed in U. S. Patent No. 4,346,227) and velostatin also referred to as synvinolin and simvastatin (disclosed in U. S. Patents Nos. 4,448,784 and 4,450,171).

Other compounds which lower serum cholesterol may do so by an entirely different mechanism than the HMG CoA reductase inhibitors. For example, serum cholesterol may be lowered through the use of bile acid sequestrants such as cholestyramine, colestipol, DEAE-Sephadex and poly(diallylmethylamine) derivatives (such as disclosed in U. S. Patents Nos. 4,759,923 and 4,027,009) or through the use of antihyperlipoproteinemics such as probucol and gemfibrozil.

U. S. Patent No. 4,759,923 mentioned above discloses that poly(diallylmethylamine) derivatives which are bile salt sequestrants may be used in conjunction with drugs which reduce serum cholesterol by mechanisms other than sequestration, such as clofibrate, nicotinic acid, probucol, neomycin, p-aminosalicylic acid or mevinolin (also referred to as lovastatin).

The use of HMG CoA reductase inhibitors in diabetics is known as indicated by the paper Yoshino G. et al, "Effect of CS-514, an inhibitor of 3-hydroxy-3-methylglutaryl coenzyme A reductase, on lipoprotein and apolipoprotein in plasma of hypercholesterolemic diabetics," Diabetes Res. Clin. Pract. (Netherlands), 1986, 2/3 (179-181). CS-514 refers to pravastatin. In addition, Garg and Grundy, "Lovastatin Therapy for Cholesterol Lowering in Non-Insulin Dependent Diabetes mellitus (NIDDM)," Clin. Res. (35, No. 3, 503A, 1987), conclude that coronary risk in NIDDM should be reduced by lovastatin therapy.

East, C.A. et al, "Treatment of Type 3 Hyperlipoproteinemia with Mevinolin," Metab. Clin. Exp. (USA), 1986, 35, No. 2, 97-98 discloses that mevinolin reduced very low density lipoproteins (VLDL) and LDL in a man with Type 3 hyperlipoproteinemia.

Sirtori, C.R. et al, "Effects of Fibrates on Serum Lipids and Atherosclerosis," Pharmac. Ther., Vol. 37, pp. 167 to 191, 1988 (G.B.), discloses that various fibrates, namely, bezafibrate, ciprofibrate, fenofibrate, gemfibrozil and clofibrate, possess lipid-lowering effects and reduce serum cholesterol and triglyceride levels.

The use of combinations of various types of cholesterol lowering drugs have been suggested in the literature. For example, Brown, W.V., "Potential Use of Fenofibrate and Other Fibric Acid Derivatives in the Clinic," Am. J. Med., Nov. 27, 1987, Vol. 83 (suppl 5B), indicates at page 85 that "in combination with other agents that lower low-density lipoprotein levels more specifically, such as the bile acid sequestrants and hydroxymethylglutaryl coenzyme A reductase inhibitors, fenofibrate may act to effect control of the triglycerides allowing management of those patients with disorders producing elevated very low-density lipoprotein and low-density lipoprotein levels."

Weisweiler, P., "Simvastatin and Bezafibrate Effects on Serum Lipoproteins and Lecithin Cholesterol Acyltransferase Activity in Familial Hypercholesterolemia," Eur. J. Clin. Pharmacol. 35(6), 1988, 579-584, indicates that "simvastatin was clearly more effective than bezafibrate in lowering LDL by enhancing its turnover, but bezafibrate had specific effects on VLDL and HDL that might be favorable in combined treatment regiments."

East, C. et al, in a letter to the Editor N. Engl. J. Med., Vol. 318, No. 1, Jan. 7, 1988, pp. 47-48, disclose that 4 patients after cardiac transplant received lovastatin (2 of which also received gemfibrozil) and developed rhabdomyolysis.

Physicians' Desk Reference, 44th Ed., 1990, page 1413 reports that severe cases of rhabdomyolysis have been associated in patients receiving lovastatin in combination with either gemfibrozil or lipid-lowering doses of nicotinic acid. "Acute renal failure from rhabdomyolysis has been seen more commonly with the lovastatin-gemfibrozil combination, and has also been reported in transplant patients receiving lovastatin plus cyclosporine."

"Fulminant rhabdomyolysis has been seen as early as three weeks after initiation of combined therapy with gemfibrozil and lovastatin, but may be seen after several months. For these reasons, it is felt that, in most subjects who have had an unsatisfactory lipid response to either drug alone, the possible benefits of combined therapy with lovastatin and gemfibrozil do not outweigh the risks of severe myopathy, rhabdomyolysis, and acute renal failure. While it is not known whether this interaction occurs with fibrates other than gemfibrozil, myopathy and rhabdomyolysis have occasionally been associated with the use of other fibrates alone, including clofibrate. Therefore, the combined use of lovastatin with other fibrates should generally be avoided."

Illingworth, D.R.; Bacon, S., Am. J. Cardiol. (60, No. 12, 33G-42G, 1987) in their paper entitled "Hypolipidemic Effects of HMG CoA Reductase Inhibitors in Patients with Hypercholesterolemia," disclose the use of 3-hydroxy 3-methylglutaryl coenzyme A (HMG CoA) reductase inhibitors such as mevastatin (compactin), lovastatin (mevinolin), simvastatin (synvinolin) and pravastatin (CS-514, eptastatin or SQ-31000) in the treatment of familial hypercholesterolemia. "In patients who do not experience adequate control with HMG CoA reductase inhibitors combination therapy with cholestyramine, colestipol, neomycin, probucol, nicotinic acid, gemfibrozil, bezafibrate, fenofibrate, ciprofibrate or clofibrate may be initiated."

Illingworth, D.R., Clin. Chem. (34,No. 8, Suppl., B123-B132, 1988), in a paper entitled "Drug Therapy of Hypercholesterolemia," discloses that drugs available for treatment of hypercholesterolemia include cholestyramine and colestipol, nicotinic acid, HMG CoA reductase inhibitors such as lovastatin, mevastatin, simvastatin, pravastatin and SRI62320; fibric acid derivatives such as clofibrate, gemfibrozil, fenofibrate, bezafibrate and ciprofibrate, probucid, neomycin, and d-thyroxin. "Combinations of 2 or 3 drugs with different mechanisms of action can be used in severe hypercholesterolemia. It is concluded that currently available drugs are capable of reducing plasma cholesterol sufficiently to alter the course of premature atherosclerosis in patients with severe hypercholesterolemia."

In some cases, use of a combination of cholesterol-lowering drugs may cause serious side effects. For example, Norman, D.J. et al, in a letter to the Editor, N. Engl. J. Med., Vol. 318, No. 1, Jan. 7, 1988, pp. 46 and 47, disclose that myolysis and acute renal failure occurred in a heart-transplant recipient receiving lovastatin in combination with nicotinic acid.

Marais, G.E. et al, "Rhabdomyolysis and Acute Renal Failure Induced by Combination Lovastatin and Gemfibrozil Therapy," Annals of Internal Medicine, Feb. 1,, 1990, Vol. 112, No. 3, pp. 228-230, report that in a single patient, "rhabdomyolysis and acute renal failure were directly attributable to the addition of gemfibrozil to lovastatin therapy. We conclude that gemfibrozil and lovastatin should be used together only with great caution."

In accordance with the present invention, a pharmaceutical combination is provided for use in treating dyslipidemia in diabetics and non-diabetics, which combination is formed of an inhibitor of the enzyme 3-hydroxy-3-methylglutaryl coenzyme A (HMG CoA) reductase which is pravastatin and a fibric acid derivative. The pravastatin will be employed in a weight ratio to the fibric acid derivative of within the range of from about 0.001:1 to about 1000:1 and preferably from about 0.05:1 to about 100:1.

In addition, in accordance with the present invention, a method is provided for treating dyslipidemia in diabetics or non-diabetics, wherein a therapeutically effective amount of the above combination is systemically, such as orally or parenterally, administered over a prolonged period.

It has been found that the combination of pravastatin and the fibric acid derivative, which works by a mechanism other than inhibiting HMG CoA reductase, is a surprising and unique concept in treating dyslipidemia in both diabetics and non-diabetics, in that it may provide additional anticholesterolemic effects over that which may be obtained using each of the components of the combination alone. Reduced levels of each of the pravastatin and the fibric acid derivative may be employed to achieve desired results, albeit with reduced side effects.

Surprisingly, the combination of pravastatin and the fibric acid derivative of the invention which is useful in treating dyslipidemia is substantially safer than use of the combination of lovastatin and fibric acid derivatives.

The term "dyslipidemia" as employed herein refers to mixed hyperlipidemia, that is, conditions of elevated cholesterol (LDL and total cholesterol) and elevated triglycerides.

The combination of the present invention is particularly effective in the treatment of dyslipidemia in diabetics.

The fibric acid derivatives suitable for use herein include, but are not limited to, fenofibrate, gemfibrozil, clofibrate, bezafibrate ciprofibrate, clinofibrate and the like.

Preferred are combinations of pravastatin with fenofibrate, gemfibrozil or bezafibrate.

In carrying out the method of the present invention, the combination of the invention may be administered to mammalian species, such as monkeys, dogs, cats, rats, humans, etc. and as such may be incorporated in a conventional systemic dosage form, such as a tablet, capsule, elixir or injectable. The above dosage forms will also include the necessary carrier material, excipient, lubricant, buffer, antibacterial, bulking agent (such as mannitol), anti-oxidants (ascorbic acid or sodium bisulfite) or the like. Oral dosage forms are preferred, although parenteral forms are quite satisfactory as well.

The dose administered must be carefully adjusted according to age, weight and condition of the patient, as well as the route of administration, dosage form and regimen and the desired result.

Thus, for oral administration, a satisfactory result may be obtained employing the pravastatin in dosages employed, for example, for lovastatin as indicated in the Physicians' Desk Reference, such as in an amount within the range of from about 1 to 2000 mg and preferably from about 4 to about 200 mg in combination with the fibric acid derivative in dosages normally for these cholesterol lowering drugs, for example, as indicated in the Physicians' Desk Reference, in the case of gemfibrizol, and clofibrate such as in an amount within the range of from about 2 mg to about 7500 mg and preferably from about 2 mg to about 4000 mg, with pravastatin and the fibric acid derivative being employed together in the same oral dosage form or in separate oral dosage forms taken at the same time.

A preferred oral dosage form, such as tablets or capsules, will contain the pravastatin in an amount of from about 0.1 to about 100 mg, preferably from about 5 to about 80 mg, and more preferably from about 10 to about 40 mg, and the fibric acid derivative in an amount of from about 2 to about 3000 mg, preferably from about 2 to about 2000 mg.

The composition described above may be administered in the dosage forms as described above in single or divided doses of one to four times daily. It may be advisable to start a patient on a low dose combination and work up gradually to a high dose combination.

Tablets of various sizes can be prepared, e.g., of about 2 to 2000 mg in total weight, containing one or both of the active substances in the ranges described above, with the remainder being a physiologically acceptable carrier of other materials according to accepted pharmaceutical practice. These tablets can, of course, be scored to provide for fractional doses. Gelatin capsules can be similarly formulated.

Liquid formulations can also be prepared by dissolving or suspending one or the combination of active substances in a conventional liquid vehicle acceptable for pharmaceutical administration so as to provide the desired dosage in one to four teaspoonsful.

Such dosage forms can be administered to the patient on a regimen of one to four doses per day.

According to another modification, in order to more finely regulate the dosage schedule, the active substances may be administered separately in individual dosage units at the same time or carefully coordinated times. Since blood levels are built up and maintained by a regulated schedule of administration, the same result is achieved by the simultaneous presence of the two substances. The respective substances can be individually formulated in separate unit dosage forms in a manner similar to that described above.

Fixed combinations of pravastatin and fibric acid derivatives are more convenient and are preferred, especially in tablet or capsule form for oral administration.

In formulating the compositions, the active substances, in the amounts described above, are compounded according to accepted pharmaceutical practice with a physiologically acceptable vehicle, carrier, excipient, binder, preservative, stabilizer, flavor, etc., in the particular type of unit dosage form.

Illustrative of the adjuvants which may be incorporated in tablets are the following: a binder such as gum tragacanth, acacia, corn starch or gelatin; an excipient such as dicalcium phosphate or cellulose; a disintegrating agent such as corn starch, potato starch, alginic acid or the like; a lubricant such as stearic acid or magnesium stearate; a sweetening agent such as sucrose, aspartame, lactose or saccharin; a flavoring agent such as orange, peppermint, oil of wintergreen or cherry. When the dosage unit form is a capsule, it may contain in addition to materials of the above type a liquid carrier such as a fatty oil. Various other materials may be present as coatings or to otherwise modify the physical form of the dosage unit. For instance, tablets or capsules may be coated with shellac, sugar or both. A syrup or elixir may contain the active compound, water, alcohol or the like as the carrier, glycerol as solubilizer, sucrose as sweetening agent, methyl and propyl parabens as preservatives, a dye and a flavoring such as cherry or orange.

Some of the active substances described above form commonly known, pharmaceutically acceptable salts such as alkali metal and other common basic salts or acid addition salts, etc. References to the base substances are therefore intended to include those common salts known to be substantially equivalent to the parent compound.

The formulations as described above will be administered for a prolonged period, that is, for as long as the dyslipidemia remains or the symptoms continue. Sustained release forms of such formulations which may provide such amounts biweekly, weekly, monthly and the like may also be employed. A dosing period of at least one to two weeks are required to achieve minimal benefit.

The following Examples represent preferred embodiments of the present invention. All temperatures are expressed in degrees Centigrade unless otherwise indicated and all mesh sizes are U.S. Standard ASTME.

Example 1

A pravastatin formulation in the form of tablets having the following composition was prepared as described below.

| Ingredient | Parts by Weight |
|---|---|
| Pravastatin | 7 |
| Lactose | 67 |
| Microcrystalline cellulose | 20 |
| Croscarmellose sodium | 2 |
| Magnesium stearate | 1 |
| Magnesium oxide | 3 |

Pravastatin, magnesium oxide and a fraction (30%) of the lactose were mixed together for 2 to 10 minutes employing a suitable mixer. The resulting mixture was passed through a #12 to #40 mesh size screen. Microcrystalline cellulose, croscarmellose sodium and the remaining lactose were added and the mixture was mixed for 2 to 10 minutes. Thereafter, magnesium stearate was added and mixing was continued for 1 to 3 minutes.

The resulting homogeneous mixture was then compressed into tablets each containing 5 mg, 10 mg, 20 or 40 mg pravastatin.

Fenofibrate tablets containing 250 mg fenofibrate are prepared employing conventional procedures containing the following additional ingredients: corn starch, ethyl cellulose, glycerin, hydroxypropyl cellulose, hydroxypropylmethyl cellulose 2910, iron oxide, lactose, magnesium stearate, microcrystalline cellulose, polysorbate 80, talc and titanium dioxide.

The pravastatin tablets and fenofibrate tablets may be administered as a combination in accordance with the teachings of the present invention to treat dyslipidemia in non-diabetic as well as in diabetic patients. In addition, the pravastatin and fenofibrate tablets may be ground up into powders and used together in a single capsule.

Examples 2

Pravastatin tablets are prepared employing conventional pharmaceutical techniques containing 20 mg pravastatin and inert ingredients employed in lovastatin tablets, namely cellulose, color, lactose, magnesium stearate and starch and butylated hydroxyanisole as a preservative as described in the 1990 PDR.

The pravastatin tablets may be employed in combination with gemfibrozil tablets containing 300 mg gemfibrozil and inactive ingredients as described in the 1990 PDR, in separate or combined dosage forms to treat dyslipidemia in non-diabetic as well as in diabetic patients in accordance with the present invention.

Examples 3

Pravastatin tablets, described in Examples 1 and 2, respectively, may be employed in combination with clofibrate tablets containing 500 mg clofibrate and inactive ingredients as described in the 1990 PDR. The pravastatin and clofibrate may be employed in separate dosage forms or combined in a single capsule form to treat dyslipidemia in non-diabetic as well as diabetic patients in accordance with the present invention.

Examples 4 to 6

Combinations of pravastatin and ciprofibrate, bezafibrate and clinofibrate may also be prepared in a manner described hereinbefore in Example 1 to 3 for use in treating dyslipidemia in non-diabetic as well as diabetic patients.

**Claims**

1. A pharmaceutical combination comprising an inhibitor of the enzyme 3-hydroxy-3-methyl-glutaryl coenzyme A (HMG CoA) reductase which is pravastatin and a fibric acid derivative.

2. The combination as defined in Claim 1 wherein said fibric acid derivative is gemfibrozil, fenofibrate, clofibrate, bezafibrate, ciprofibrate or clinofibrate.

3. The combination as defined in Claim 1 wherein said fibric acid derivative is fenofibrate, gemfibrozil or bezafibrate.

4. The combination as defined in Claim 1 wherein pravastatin is present in a weight ratio to the fibric acid derivative of within the range of from about 0.001:1 to about 1000:1.

5. The combination as defined in Claim 1 wherein the fibric acid derivative is fenofibrate.

6. The combination as defined in Claim 1 wherein the fibric acid derivative is gemfibrozil.

7. The combination as defined in Claim 1 wherein the fibric acid derivative is bezafibrate.

8. A pharmaceutical combination comprising pravastatin and a fibric acid derivative for treating dyslipidemia, in a patient in need of such treatment.

9. The combination as defined in Claim 8 wherein said fibric acid derivative is gemfibrozil, fenofibrate, clofibrate, bezafibrate, ciprofibrate or clinofibrate.

10. The combination as defined in Claim 8 wherein the patient is a diabetic.

11. An anti-dyslipidemia composition comprising a combination as defined in Claim 1, and a pharmaceutically acceptable carrier for each component to form separate dosage forms or to form a single dosage form.

12. A pharmaceutical combination as defined in Claim 1 for treating dyslipidemia in diabetics.

13. The combination as defined in Claim 12 wherein said fibric acid derivative is gemfibrozil, fenofibrate, clofibrate, bezafibrate, ciprofibrate or clinofibrate.

**European Patent Office**

# EUROPEAN SEARCH REPORT

Application Number

**EP 91 10 6054**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 276 807 (WARNER-LAMBERT CO.)<br>* Pages 10-12 completely (claims 1-14) *<br>– – – | 1-13 | A 61 K 31/19<br>A 61 K 31/365 //<br>(A 61 K 31/365 |
| A | CHEMICAL ABSTRACTS, vol. 112, no. 17, 23rd April 1990, page 52, abstract no. 151577a, Columbus, Ohio, US; J.M. BARD et al.: "Fibrates, sequestrant resins and HMG CoA reductase inhibitors and lipoprotein particles",<br>& PROTIDES BIOL. FLUIDS 1989, 36 417-21<br>* Abstract *<br>– – – – – | 1-13 | A 61 K 31:19 ) |

TECHNICAL FIELDS
SEARCHED (Int. Cl.5)

A 61 K

The present search report has been drawn up for all claims

| Place of search | Date of completion of search | Examiner |
|---|---|---|
| The Hague | 01 August 91 | BRINKMANN C. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

&: member of the same patent family, corresponding document